# EUROPEAN PATENT APPLICATION

(11) **EP 3 330 224 A1**
(43) Date of publication of application: **06.06.2018**
(21) Application number: 17204692.2
(22) Date of filing: 30.11.2017
(51) Int. Cl.: C01B 33/40, A61K 6/00, A61K 8/00

(54) **A METHOD FOR PRODUCING A MODIFIED SAPONITE, THE MODIFIED SAPONITE AND AN INTER-PACKET WATER OBTAINED BY SAID METHOD, A COMBINATION COMPRISING THEM, USE OF THE MODIFIED SAPONITE AND THE COMBINATION AND PRODUCTS COMPRISING THEM**

(30) Priority: 30.11.2016 PL 41964316
(71) Applicant: Eko Saponit sp. z o.o., 10-273 Olsztyn (PL)
(72) Inventor: Bozhko, Anatoliy, Kiev (UA)
(74) Representative: Walkiewicz, Sebastian Rafal

(57) **Abstract**

The invention relates to: a method for producing a modified saponite, the modified saponite and an inter-packet water obtained by said method, a combinations comprising them, use of the modified saponite and the combinations and products comprising them. The invention is applicable in various technical fields, and especially in medicine, cosmetic industry, food industry and agriculture.

## Description

### Field of invention

The subject of the invention is a method for producing a modified saponite, the modified saponite and an inter-packet water obtained by said method, combination comprising them, use of the modified saponite and the combination and products comprising them. The invention is applicable in various technical fields, and especially in medicine, cosmetic industry, food industry and agriculture.

### State of the art

Saponite is trioctahedral phyllosilicate belonging to the smectite group. This mineral has been first described in 1840 by Swedish mineralogist Lars Fredrik Svanberg. Saponite is a white, yellow, red or green clay mineral having a soft structure and plasticity. Usually it is present in veins and basalt vesicles and in serpentinite. This mineral can be found *inter alia* in Great Britain (Cornwall, Skye Island), Sweden (Svärdsjö), Czech Republic (Caslaw), Poland ( 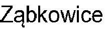 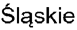 ), RPA (Krugersdorp), USA (Michigan, Minnesota, Utah, California, Arizona) and Canada (Ontario) (http://rruff.info/doclib/hom/saponite.pdf). Rich deposits are also found in Ukraine (Khmelnytskyi Oblast).

The chemical formula of the saponite (http://www.mindat.org/min-3528.html) is:

Ca_{0.25}(Mg,Fe)₃((Si,Al)₄O₁₀)(OH)₂ · nH₂O.

Empirically developed formula (http://www.webmineral.com/data/Saponite.shtml#.WBMb8smLV0s) is:

Ca_{0,1}Na_{0,1}Mg_{2,25}Fe²⁺_{0,75}Si₃AlO₁₀(OH)₂•4(H₂O).

The formulas presented above represent the composition of the saponite in an inaccurate manner, as it may contain plurality of admixtures of other compounds and elements. According to reports in literature the saponite composition consists also of SiO₂, TiO₂, Al₂O₃, Fe₂O₃, FeO, MnO, NiO, MgO, CaO, Na₂O, K₂O, H₂O⁺, H₂O⁻ and P₂O₅.

Additionally, the composition may vary depending on the origin of mineral. For example, the saponite from deposits in Czech Republic has a following, experimentally developed formula:

(Ca_{0,16}Mg_{0,05}Na_{0,04}K_{0,03})_{S=0,28}(Mg_{2,37}Fe²⁺_{0,32}Fe³⁺_{0,21}Al_{0,10})_{S=3,00}(Si_{3,16}Al_{0,84})_{S=4,0}O₁₀(OH)₂•n(H₂O)

while the formula for the saponite from deposits in California is:

(Ca_{0,19}Na_{0,16}K_{0,02})_{S=0,37}(Mg_{2,61}Al_{0,15}Fe2⁺_{0,06})_{S=2,82}(Si_{3,77}Al_{0,23})_{S=4,0}O₁₀(OH)₂•n(H₂O).

Due to its structure, the saponite exhibits internal crystalline swelling in water and many organic compounds, as well as well-defined cation-exchange properties. Furthermore, according to the prior art, saponite exhibit potent sorption properties.

The prior art describes numerous applications of this mineral in various fields of technology, including cosmetology, medicine, agriculture, food industry and others.

Publications by M.Pozo et. al., "Clay and non-clay minerals in the pharmaceutical and cosmetic industries" Part 1 and 2, Applied Clay Science, September 2009 and February 2010, describe several applications of the saponite and similar minerals in the field of cosmetics and medicine, both as active and auxiliary agents. Authors of the publication *inter alia* indicate that the saponite (as well as other smectites) may be used as disintegrating agent, diluent, binder, emulsifier, thickener, anti-caking agent, taste correcting agent and component influencing release of active substances. Furthermore, authors stated that natural saponite is also used in spa centres for therapeutic purposes due to its physical properties (softness, particle size, rheological properties, high absorption capacity) and cation-exchange properties. As an active substance, the saponite, as well as other smectites, may be used as acid-neutralizing agent, digestive tract protecting agent, anti-diarrheal, protective dermatological agent, component of cosmetic creams, powders and emulsions.

### Use of saponite in cosmetology

Document GB1425177 discloses a clay composition comprising smectite clay (e.g. saponite) and fatty acid, intended for mixing with detergents.

Document JP2000159630 discloses a cosmetic composition for application on skin comprising clay (e.g. saponite) and succinoglucan.

Document JPS6081124 describes a cosmetic composition for application on skin, comprising UV radiation absorbing agent, clay (e.g. saponite) and water.

Document JPH10330211 describes a cosmetic composition for application on skin comprising swelling smectite clay (e.g. saponite) having a mean particle size in the range of 1-5000 nm, preferably below 1000 nm, and purity greater than 90%, and ester of polyglycerol and fatty acid. The method for obtaining the indicated particle size of saponite is not described.

JPH0632721 discloses a formulation for application on skin comprising vitamin A and one or two clays (e.g. saponite).

JP2000247893 discloses a bathing agent having antimicrobial properties, comprising saponite.

Document JPH0570341 discloses bathing composition comprising smectite clay (e.g. saponite) and oil component/humectant (e.g. plant or animal oil, ester oil, higher alcohol, hydrocarbon oil, etc.) and inorganic salts.

Document JP2000355515 discloses a cosmetic composition for use in atopic dermatitis, comprising powdered coral, hydrous silicate (e.g. saponite), salt of benzoic acid and water.

JP2002037723 describes a sheet shaped cosmetic product comprising non-spherical particles of powdered aluminosilicate (e.g. saponite) intercalated on unwoven fabric.

Document JP2005029534 discloses a two-component composition for application on hair (e.g. in shampoo) comprising (i) smectite clay (e.g. saponite) and (ii) cationic surfactant and silicate compound.

JP2012102056 discloses a hair styling agent in the form of gel comprising silicic anhydride, smectite (e.g. saponite) and water.

Document JPH01287011 discloses a composition for use in lipstick, comprising colorant and swelling clay (e.g. saponite).

JPH03190810 describes a quick-drying clay composition comprising swelling clay (e.g. saponite) and emulsion of resin (e.g. synthetic) for use in cosmetology or as a substrate for producing synthetic rubber.

JPH04173725 describes a foamy depilatory agent comprising calcium thioglycolate and at least one swelling clay (e.g. saponite).

Document JPH09227338 discloses a cosmetic make-up powder composition comprising swelling clay (e.g. saponite) and a powder subjected to hydrophobic treatment.

KR100789345 discloses a solid composition of w/o emulsion type, comprising saponite as an inorganic gelling agent.

Document KR20130126292 describes a perfumed cosmetic composition comprising a thickener being a mixture of inorganic compounds, including saponite.

### Use of saponite in medicine and pharmacy

Experiments indicated use of saponite as an enterosorbent during viral and bacterial intestine disorders, indented for removing heavy metal salts, radionuclides and pathogenic microbes (e.g. polio viruses, type A hepatitis viruses, *Escherichia coli,* staphylococci and others) from the human body. The effectiveness of saponite acting as enterosorbent is higher than the activity of activated carbon. Furthermore, the activated carbon may damage the surface of digestive tract mucosa and it contains synthetic contaminants (benzo-a-piren and dioxins).

US2007/231412 discloses use of saponite for deactivating viruses in digestive tract.

International patent application WO2009/108867 discloses a method for preventing circulatory system diseases related to atherosclerosis by administering material being a phyllosilicate (e.g. saponite) in order to treat atherosclerotic lesions and to absorb cholesterol.

Document CN1298719 describes a medicament for treating kidney stone disease, comprising herbal components, powdered saponite and potassium chloride.

JP2000016941 discloses an agent for preventing hay fever, comprising swelling clay e.g. saponite.

Document JP20002316925 describes an agent for alleviating symptoms of hay fever, comprising lower monohydric alcohol and swelling clay (e.g. saponite).

Document RU2462243 discloses an agent for treating alcohol withdrawal syndrome, comprising glycine and saponite.

Document JPS5867623 describes a medicine for treating dermatophytosis, being a mixture of powdered clay e.g. saponite and salicylic acid or phenol.

In medical/pharmaceutical applications, saponite is also used as auxiliary agent.

KR20100042354 discloses a transdermal composition for preventing vomiting, comprising antiemetic medicament disposed between layers of a swelling clay (e.g. saponite).

Document KR20130003099 describes antifungal composition comprising itraconazole, polysaccharide and a swelling clay (e.g. saponite).

### Use of saponite in agriculture and breeding

Incredible effect of natural saponite used in agriculture, used both with respect to soil cultivation as well as animal husbandry, is known in the prior art.

The conducted experiments showed that saponite added to feed intended for livestock (i.a. pigs, cattle, poultry) provides body weight growth increase of about 30% (pigs), about 11% (cattle), about 5 % (poultry - broilers) with respect to control groups fed with feed without such an admixture.

The conducted experiments confirmed also absolute lack of any toxic influence of saponite on fed animals. The advantageous micro- and ultra-microelements comprised in saponite are well absorbed, promote the increase of haemoglobin levels in blood and improve the nitrogen utilization. The conducted experiments confirmed also that it is safe to utilize meat of animals fed with such enriched feed in human food.

Additional effect of using the saponite admixture is reduction of financial outlays related to feeds.

Natural saponite and compositions comprising saponite with phosphoric acid and ammonia proved to be an effective preservative for feed, especially sugar pomace, green feed, alfalfa, etc. Saponite used as a feed preservative significantly reduces the losses of root crops during storage.

The experiments confirmed also the high effectiveness of saponite as a land improving agent for sandy soils and podzols. Field experiments confirmed that after using saponite, corn yield for silage, oats yield and winter wheat yield increased significantly. As a result, use of saponite allows for cultivation of winter wheat on sandy soils, which is not a typical practice. Furthermore, it was observed that saponite remains in soil for up to 5 years after introduction. For comparison, other mineral fertilizers remain active for a maximum period of one season.

Effective action of clays with respect to sandy soils and podzols is justified, among others, by:
a) high content of magnesium, which varies between 9 and 11 % (based on magnesium oxide), while sandy soils suffer from a severe magnesium deficit,
b) high content of potassium fertilizer - one ton of saponite contains from 10 to 20 kg of potassium oxide K₂O, which is absolutely necessary in an amount of 45-60 kg/ha in order to increase fertility of degraded and leached chernozems, podzols, slightly sandy and clay soils and peat bogs.

For plants with increased demand for potassium (sugar beets, potatoes, sunflower, linen, root crops, legumes, vegetables, fruit and berry plants, perennial grasses and others) saponite is a land improving agent of high effectiveness.

Introduction of saponite into soil in defined doses increases the content of active mineral fraction, having pronounced ion exchange properties. With the increase of soil absorption capacity, its fertility also increases significantly, especially with the increase of elements which are exchanged and absorbed by plants. Especially during second and third year after application, it leads to a much greater accumulation of mobile potassium and magnesium in soil than in case of using dolomite flour. In case of light soils it provides a great increase in crop yield.

Furthermore, saponite contains elements such as calcium, magnesium, potassium, sodium, boron and others, which are very scarce in sandy soils. Their percentage in relation to magnesium content is relatively low, but after application of saponite in indicated doses, soil receives significant amount of said elements in a form which is easily absorbed, which is undoubtedly important for improving their balance.

Natural saponite is also used as a soil deoxidizer and its effectiveness is even better than the effectiveness of traditionally used calcareous flour. The effectiveness of saponite in comparison to calcareous flour is especially pronounced (two times greater) during the second year after application.

Saponite is also used during recultivation of soils contaminated with radioactive isotopes. The experiments showed saponite effectiveness in removing cerium 137 isotope. Saponite may also be used in detoxification of post-technological and post-industrial soils.

Document CN105076850 discloses feed for meat poultry comprising plant components and powdered saponite.

FR2510876 and IT1168179 describe feed for ruminants comprising swelling porous mineral (e.g. saponite).

Document RU2530996 discloses feed for sheep enriched with saponite suspension irradiated by UV lamps.

UA95312 discloses feed for coypus comprising corn, barley, fish meal, chalk, salt, oats, soy meal and natural saponite.

US5165946 discloses a method for purifying dry feed from mycotoxins by mixing it with multilayer silicate (e.g. saponite), which deactivates mycotoxins.

Document UA81037 discloses use of saponite as a sorbent for poultry feed.

KR20130039404 discloses a composition used as a feed supplement, comprising natural or synthetic inorganic layered compound (e.g. saponite) and phytoncide.

Document UA36826 discloses a method for carp breeding employing deposition of natural clay material (e.g. saponite), distillery waste (seeds) and by-products from grain production on the bottom of a basin.

UA47689 and UA86527 describe methods for improving feed with use of a mixture of saponite flour, vitamin D3 produced by UV irradiation and water.

RU2379872 discloses capsules made from water soluble polymer, comprising seed, nutrients, mineral fertilizers (e.g. saponite) and microelements.

Document UA13101 discloses a preservative for silage and haylage, comprising sodium chloride and natural mineral (e.g. saponite).

UA26051 discloses a bioactive fertilizer comprising organic waste of anthropogenic origin, organic carbonaceous carrier, manganese sulphate, exoenzymes of specific microbiocenosis, carbamide, superphosphate, potassium fertilizers, ameliorants in the form of gypsum or phosphogypsum, lime or marl, or saponite, perlite, ceolite, microelements and water.

UA54410 discloses a method for purifying seeds from fungi with use of potassium permanganate and a bath comprising saponite flour.

Document UA61935 describes a method for grain crops with use of powdered mustard and saponite flour, in order to fight fungi and bacteria.

JP2000139203 discloses an agent for use on cuttings, comprising a mixture of porous agent and smectite mineral (e.g. saponite).

Document US5763364 discloses use of saponite as an auxiliary agent for production of concentrates of plant protecting agents.

Document EP 0 425 729 describes pesticide concentrate comprising smectite flour (e.g. saponite) as a thickener.

GB 970 579 discloses pesticide concentrate comprising saponite as a suspending agent.

Document JPH04327502 discloses pesticide concentrate comprising saponite as an auxiliary agent.

Document GB1356672 describes an agent for eradicating rice stem bores comprising saponite as a carrier.

Natural saponite is also used in food industry.

UA89371 discloses a device for enriching drinking water with minerals and vitamins, using powdered saponite in the form of suspension.

International patent application WO9516024 describes a method for producing drinks including addition of thermally treated (in the temperature range of 550 - 1000°C for maximum period of one hour) phyllosilicate (e.g. saponite), mixing and leaving the mixture until the reaction is complete, and then filtrating.

Document JPH09208404 describes an acaricidal composition comprising 2-(acetyloxy)-3-dodecyl-1,4- naphthalenedione, smectite (e.g. saponite), anionic or non-ionic surfactant and thickener.

### Use of sorptive properties of natural

GB1482930 describes a method for removing metal ions from solutions using cation-exchange inorganic substrate made from e.g. smectite such as saponite.

Document UA45002 describes a method for purifying water from metal ions by filtration through a clay such as natural saponite.

UA82945 describes an autonomous installation for water treatment, using, among others, mineral fillers made from saponite.

US2005082229 discloses a composition for purification of water from phosphates, containing i.a. alum and smectite mineral (e.g. saponite).

US2006016757 describes a method for purifying water from hexavalent chromium ions, including reacting water with natural saponite clay containing iron at a temperature in the range of 50-200°C for 1-3 hours.

International patent application WO2016092123 discloses a method for the production of absorbents based on mineral clays (e.g. saponite) including dispersing and delaminating of the starting material, impregnating monolayers in a reactive solution containing metal ions, filtering and washing, drying and calcination, and finally grinding. Such absorbents are used for water purification.

UA68206 discloses a method for solidifying liquid, alkaline radioactive contaminants with the use of natural adsorbent (e.g. saponite) and cement as a binder.

Document UA90891 describes filters containing a blend of a powdered metal and a natural sorbent - saponite.

### Other uses of saponite

Document JP2000345024 discloses a composition having plastic properties, comprising saponite, polyethylene oxide and water.

JP2008050226 discloses a heat and water resistant flexible, thin-film clay, containing saponite and silicone oil.

Document JPS6350311 describes a filling agent, e.g. for the production of rubber and plastics and for the production of cosmetics, containing spray-dried spherical clay particles (e.g. saponite) in a suspension in an aqueous solvent.

US5165946 describes a bleaching agent containing an activator - ammonium nitrile and a phyllosilicate (e.g. saponite).

Document CN102757061 describes a method for the production of molecular sieves from saponite by dispersing a raw material in the water, adding a surfactant, heating (30-90°C) and synthesizing of the molecular sieve.

GB795051 describes a method for modifying a hydrophilic solid (e.g. a saponite) with an organic isocyanate or isothiocyanate.

Methods for increasing the activity of saponite or changing its properties are known in the prior art. These methods include treating the mineral with various chemical reagents in order to exchange cations present in the saponite crystal structure to ones which exhibit desired properties. A frequently used type of activation is an acid or alkaline activation. Another way to modify the natural saponite is to bind the desired functional groups to its surface.

JPS6461310 discloses the modification of the saponite by the action of specific salts of inorganic acids followed by drying and firing.

Document JP2000247893 discloses an antibacterial agent for application on the skin, containing saponite chemically treated with strychnine sulfate.

EP2431043 discloses a method for reducing the solubility of cholesterol in the digestive tract using the administration of a protonated saponite.

UA54826 discloses a method for purifying water from heavy metal ions by filtration through a sorbent - saponite activated with hydrochloric acid.

UA64424 describes a method for purifying water from organic contaminants by filtration through a suspension of saponite activated with hydrochloric acid.

UA74752 discloses a method for purifying water from metal ions using a sorbent suspension - a natural saponite stabilized with an alkaline solution.

Document UA91147 describes a sorbent based on magnetite and acid-modified saponite clay.

Document US5922206 discloses a method for purifying water from insoluble petroleum compounds using adsorbing clay (e.g. acid-activated saponite) an then polyvinylpyridine adsorbent.

PL201385 describes a method for producing a swellable phyllosilicate, including alkaline activation of a phyllosilicate (e.g. saponite), drying and grinding thereof. The modified silicate thus obtained is used, *inter alia,* as an agent for controlling the rheological properties and stability of powdered, liquid or pasty media.

GB664830 discloses a method for modifying clay (e.g. saponite) by bonding alkyl or aryl radicals on the clay surface with onium compounds.

US2013095318 discloses a method for the production of particles of controlled size, e.g. saponite particles, comprising several variants:
- particle size reduction (grinding), extraction (sieving, dust removal in a cyclone, filtration),
- particle size reduction (wash mill), extraction (sieving, dust removal in a cyclone, filtration),
- particle size reduction (grinding), extraction (centrifugation, decanting, filtration),
- particle size reduction (wash mill), extraction (centrifugation, decanting, filtration),
- particle size reduction (grinding), extraction (centrifugation, decanting, filtration), second extraction (centrifugation, decanting, filtration),
and further steps of chemical treatment, intercalation and introduction of particles into a plastic/ceramic matrix.

Products sold by Ukrainian company Veles (www.saponit.com) under name SAPROKORM are also known. The products indicated on the mentioned webpage include fertilizer, additives for feed, construction and detoxification products. Medical applications of said mineral are also indicated. The method of saponite treatment used by Veles company differs significantly from the method of the present invention. The natural saponite treatment is conducted on a wooden substrate in a very high temperature, which results in degradation of many important components of said mineral. The grinding/comminution techniques used by Veles company (including mills utilized in milling of clay materials used for porcelain production) do not allow for obtaining grains of a very small size - the smallest obtained size is 500 µm.

### Object of the invention

In the view of described prior art there is a necessity for developing a method for treatment of natural saponite, which would allow for intensification of advantageous properties of this mineral without employing chemical reagents, complicated equipment and processes. Surprisingly, method of the invention based only on mechanical and thermal treatment of natural, chemically untreated saponite allows for reaching this goal.

### Subject of the invention

The subject of the invention is a method for producing modified saponite comprising steps of:
a. providing a raw saponite,
b. initial comminution of the raw saponite,
c. drying of initially comminuted saponite with simultaneous secondary comminution, and inter-packet water separation in a temperature up to 65°C,
d. sieving a dried saponite using at least one screen to obtain at least two fractions,
e. finishing treatment to obtain at least one fraction of modified saponite having mean particle size in the range from about 0,6 µm to about 100 µm.

Preferably, the raw saponite is initially comminuted in step b) to obtain grains having a mean size in the range from about 1 to about 15 mm, preferably from about 2 to about 10 mm, more preferably from about 3 to about 8 mm, and most preferably about 5 mm.

Preferably, the drying step is conducted in a drum dryer, in which the moving saponite undergoes abrasive treatment due to the mutual friction between saponite particles, and optionally due to friction against friction elements located inside the drum of the dryer.

Preferably, during step c), a drying stream of hot air is used flowing co-currently or counter-currently relative to the direction of the flow of the dried saponite.

Preferably, drying in step c) is conducted for a period from about 60 to about 180 minutes.

Preferably, during step c) evaporated inter-packet water is discharged from the drying device.

Preferably, in step d) a set comprising at least three screens is used, providing at least three fractions having mean particle size of less than about 250 µm, less than about 500 µm and less than about 5 mm, respectively.

In another preferred embodiment, in step d) a set comprising at least three screens is used, providing at least three fractions having mean particle sizes of less than about 300 µm, less than about 1000 µm and less than about 5 mm, respectively.

Preferably, only one fraction obtained in step d) having the largest mean particle size, is directed to step e).

Preferably, dried, sieved raw material is further grinded and separated in step e) to obtain at least two fractions.

Preferably, fractions of modified saponite comprising particles having mean particle size of 0,6 µm, about 0,8 µm, about 1 µm, about 5µm, about 10 µm, about 15µm, about 20 µm, about 25 µm, about 30 µm, about 40 µm, about 45 µm, about 50 µm, about 55 µm, about 60 µm, about 65 µm, about 70 µm, about 75 µm, about 80 µm, about 85 µm, about 90 µm, about 95 µm and about 100 µm, most preferably about 1 µm, about 20 µm, about 50 µm, about 80 µm or about 100 µm, are obtained in step e).

Preferably the dried, sieved raw material additionally undergoes a finishing treatment in step e) using an assembly comprising cyclone separator and bag filter, to obtain a saponite fraction having mean particle size in nanometer range.

Another subject of the invention is a modified saponite obtained by the method of the invention.

Another subject of the invention is an inter-packet water obtained in the step c) of the method of the invention.

Another subject of the invention is a combination comprising the modified saponite of the invention and the inter-packet water of the invention.

Preferably, the combination comprises 0,1-99,9 % of the modified saponite and 0,1 - 99,9% of the inter-packet water.

Another subject of the invention is use of the modified saponite of the invention or the combination of the invention as a therapeutic product, a cosmetic product, a food product, an agricultural product, a sorption product or a construction product.

Another subject of the invention is a product comprising the modified saponite of the invention or the combination of the invention and optionally at least one additional active component and/or auxiliary components.

Preferably, the product is a therapeutic product, a cosmetic product, a food product, an agricultural product, a sorption product or a construction product.

Preferably, the product of the invention is
the cosmetic product selected from group comprising a balsam, a lotion, a shampoo, including anti-dandruff shampoo, a gel, a peeling, a mask, a formulation for applying on the skin, a bubble bath, a cream, a milk, a conditioner, a foam, a hair mask, a pomade, a wax, a hair oil, a gel, a paint or a serum for hair,
the therapeutic product selected from group comprising a composition for treating gums, an agent for treating alcohol withdrawal syndrome, a medicament for treating skin diseases, anti-psoriatic medicament, a medicament for treating digestive tract diseases, a medicament for treating the circulatory system diseases, a medicament for treating poisoning, a medicament for use in case of fatigue and exhaustion, a medicament for treating diseases of bones and/or joints, a medicament for treating edema, a medicament for the treatment of oral cavity diseases, an anti-allergic medicament, an anti-inflammatory medicament, an antidiabetic medicine, a medicament for treating diseases of the nervous system, a medicament for treating respiratory system diseases, an anti-asthmatic medicament, an urological medicament, an intestinal sorbent, an antiviral medicament, an antibacterial medicament and a balneological medicament,
the agricultural product selected from group comprising a mineral feed, a feed additive, a feed preservative, a premix, a mineral and vitamin supplement for animals, a crop support, a plant protection product, a crop preservative, a soil conditioner, a fertilizer, a mineral fertilizer and a soil deoxidizer,
the adsorption product selected from group comprising a detoxifying agent, an agent for soil recultivation, filter cartridge,
the food product selected from group comprising confectionery and bakery products,
the construction product selected from group comprising thermal isolation and waterproofing elements, fillers and LECA.

### Advantages of the invention

The modified saponite obtained by the method of the invention, as well as the combination comprising the modified saponite and inter-packet water, exhibit surprisingly advantageous properties in comparison to natural, untreated saponite, as well as in comparison to known formulations containing saponite treated mechanically/thermally according to known procedures. The advantageous properties have been obtained thanks to the steps of treatment of the natural saponite, and in case of combination also thanks to the properties of the activated inter-packet water, which exhibits better properties in terms of effectiveness of action with respect to human body and biological subjects than the magnetically activated water.

Furthermore, method of the invention utilizes only mechanical and thermal treatment without using any chemical reagents. As a result, the assembly of the invention does not contaminate the environment.

According to the observations made by the inventors, in case of administration to the skin it is possible to achieve penetration of saponite constituents deep into the skin up to a depth of 3 cm.

### Detailed description of the invention

Method of the invention consists of series of subsequent treatment steps, which will be described below in detailed manner.

### Step A - Providing the raw material

Usually, the starting material is a saponite obtained directly from deposits, comprising portions having mean size from 3 to 50 cm. Usually, the saponite is drawn out using an excavator and transported to store via road transport. Obviously, the raw material may be drawn out using any method known in the prior art and may contain portions having size beyond the range specified above. The saponite portions may have various water content depending on the weather and season.

In the first step of the method, the raw material from mine or storage is introduced into the grinding device using loading means. The loading means may be any device known in the prior art, used for feeding raw materials having the above described form to a treatment installation. For example, such loading means may include, among others, devices such as charging hoppers, feeders, dispensers, conveyors, e.g. belt conveyors, screw conveyors, etc., as well as any combination of such devices. A person skilled in the art would easily select a set of devices adapted to the given process conditions (e.g. the form of the raw material, i.e. portion size and water content, available spatial conditions, type of devices used for treating the raw material in later steps of the method, user requirements, etc.).

### Step B - Initial comminution of the raw saponite

The raw material fed by the feeding means is introduced to the step of initial comminution. This step is conducted in order to comminute the raw saponite into portions, which may be effectively treated in the subsequent steps of the inventive method. This step is conducted using a comminuting device.

The comminuting device may be any device known in the art, which is used for comminution of a raw material having consistence typical for clay minerals such as saponite. For example, such comminuting devices may *inter alia* include crushers, such as jaw crushers, impact crushers, cone crushers, roll crushers and hammer crushers. The selection of the device should be adapted by a skilled person to the given process conditions (e.g. the form of the raw material, i.e. portion size and water content, available spatial conditions, type of devices used for treating the raw material in later steps of the method, desired size after the step of initial comminution, user requirements, etc.).

The basic criterion of the device selection is the size of particles on the output of the initial comminution step. For example, in the preferred embodiment of the inventive method, in the third step of the method - drying with simultaneous secondary comminution, the provided raw material should contain particles having mean size not larger than 5 mm. In such case, it is preferable to use the comminuting device which allows for controlling the size of particles obtained after the comminution. A crusher comprising two rotors with crushing discs arranged on shafts with spacing which allows for passing particles having given maximum size - in this case 5 mm, may be used for that purpose.

### Step C - Drying with simultaneous secondary comminution

After completing the step of initial comminution, initially comminuted raw material is fed to the drying step using transporting means. The transporting means may be any devices known in the prior art, which are used to transport raw material between different steps of technological processes. For example, such transporting means may include *inter alia* devices such as feeders, conveyors, e.g. belt conveyors, screw conveyors, dispensers, charging hoppers etc., as well as any combination of such devices. A person skilled in the art would easily select a set of devices adapted to the given process conditions (e.g. available spatial conditions, type of devices used for treating the raw material in the later steps of the method, user requirements, etc.).

During the drying step, the initially comminuted raw material undergoes the drying and secondary comminution processes. Simultaneously to the drying process, process of evaporation of the inter packet water occurs, which is one of the products of the method of the invention, and which is discharged out of the drying device.

The step of drying with simultaneous secondary comminution may be conducted in any drying devices known in the prior art. Construction and type of the drying device may vary depending on the user requirements, treated material, spatial restrictions, time and temperature required for drying, planned process throughput, water content of the raw material, as well as planned water content of the saponite at the output of this step etc. The key criterion for the selection of the drying device is capability of conducting drying and secondary comminution processes at the same time, i.e. preferred are those drying devices comprising not only means for drying step, but also which allow for installation of means used for secondary comminution. Preferably, step of drying is conducted in the drum dryer.

During the drying step, initially comminuted material is guided inside the drying device using any conveying means known in the art, preferably using conveyor. It is important that the conveyor not only displaces the material inside the drying device but also allows for friction interactions between the particles of the displaced material, so that the secondary comminution may occur during drying. In such an embodiment of the invention, the conveyor acts as secondary comminution mean. Most preferably, the conveyor is a screw conveyor.

In order to strengthen the secondary comminution occurring during the drying step, the drying device may be equipped with a stationary friction elements. For example, such friction elements may have form of plates, vanes, pins, profiles or baffles, located inside of the drying device such as drum dryer. Preferably, the friction elements may be mounted to the internal walls of the drying device. While the material is guided through the drying device, particles of the material interact with said elements and the comminution process is additionally intensified.

The drying device used in this step of the inventive method should also be selected so that it would allow for installation of means for discharging the inter-packet water. Means for discharging the inter-packet water may have any configuration which allows for discharging the inter-packet water in the form of steam (e.g. at least one conduit located in the upper part of the drying device), converting it into the liquid (e.g. condenser) and storing it (e.g. reservoir). Means for discharging the inter-packet water may be an integral part of the drying device, or it may be an external assembly of the devices connected to the drying device using conduits for transporting the inter-packet water in the form of steam. The water separated in this step is named herein as "inter-packet water of the invention".

Inventors noticed that especially advantageous technical effects are achieved when the drying device is a cylindrical drum dryer having length of 9 m, equipped with the screw conveyor and the friction elements in the form of plates located on the internal wall of the dryer.

Drying temperature should be selected in order to ensure effective drying of the raw material and separation of the inter-packet water and simultaneously to ensure that the treated saponite is not deprived of any advantageous properties, which is a typical problem in the high temperature methods known in the prior art. The present inventors surprisingly noticed that especially preferred upper limit for temperature, which can be used in the method of the invention is 65°C. It should be clear for a skilled person that the process may be conducted in lower temperature and its selection will depend, among others, on the planned throughput of the drying step. Hence, drying step may be conducted in temperature ranges such as about 40-65°C, 45-60°C, 50-55°C, and especially in temperature equal to about 40, 45, 50, 55, 56, 57, 58, 59, 60, 61, 62, 63, 64 and 65 °C, optionally as well as in lower temperatures, if other process parameters allow it.

Heat used to dry the raw material may be provided by any heating means providing planned temperature value inside the drying device and in the same time ensuring uniform and repeatable drying of the raw material. For example, the heating means may be electrical heating means or means providing heat based on combustion process (e.g. gas, liquid or solid fuel). Thermal energy produced by the heating means may be introduced into the drying device using means emitting thermal energy, for example heating plates having various shapes and localization. In order to ensure uniform heating of the raw material guided within the heating device, mentioned means emitting thermal energy should be uniformly disposed inside of the drying device. The operation of the means emitting thermal energy may be supported by heat distribution means such as blowers, fans, etc. Mentioned heat distribution means may force the circulation of the warm air co-currently or counter-currently to the direction of the moving of raw material in the drying device.

Alternatively, drying process may also be conducted in the drying device equipped with jacket in which a heating medium (e.g. liquid or gaseous), fed from the heating device, circulates. In such a situation, heat is emitted by internal walls of the drying device, which act as a mean for emitting thermal energy. In this case, the circulation of the hot air may be also supported by heat distribution means as mentioned above.

A skilled person would easily select heating means, means emitting thermal energy and heat distribution means, adapted to the given process conditions (e.g. type of heating device, available spatial conditions, temperature and time range used in the drying step, user requirements, etc.).

In a preferred embodiment, heating means constitute a boiler fired with fuel (e.g. coal, oil or gas) and heating plates disposed inside the drying device (e.g. drum dryer), supported by the fan forcing the circulation of hot air.

Duration of the drying step depends on the water content of the raw material introduced into the drying device, water content required on the output of the drying step (water content of 8-10% is usually desirable), as well as on the drying temperature. Usually, period of drying is from about 1 to about 3 hours, preferably 2 hours.

Mean size of the raw material particles after the completion of the drying step is equal or lower than the mean size of particles fed into the drying step, and usually it is lower.

It should be understood that the drying device is equipped with sensors allowing for monitoring of the process parameters (temperature, water content, speed of material displacement, etc.), allowing for correction of said parameters if needed. For example if the humidity sensor shows that the water content in the product at the output of the drying step is beyond the set range, it is possible to for example increase/decrease the drying temperature and/or decrease/increase the speed of material transportation, respectively. Such operation may be conducted manually or automatically by control devices e.g. using a computer equipped with appropriate software.

### Step D - Sieving of the dried raw material

After completion of the drying step, the dried and comminuted raw material is transported to the sieving step using transporting means. The transporting means may be any device known in the prior art, which is used to transport raw material between different steps of technological processes. For example, such transporting means may, among others, include devices such as feeders, conveyors, e.g. belt conveyors, screw conveyors, dispensers, charging hoppers etc., as well as any combination of those devices. A person skilled in the art would easily select a set of devices adapted to the given process conditions (e.g. available spatial conditions, type of devices used for treating the raw material in later steps of the method, user requirements, etc.).

The sieving step is conducted in order to separate fractions of dried saponite having mean particle size as defined by user. This step is conducted using sieving means, preferably comprising a set of screens. The set of screens contains a number of screens selected according to user requirements in order to obtain required number of fractions. Mesh sizes of the screens are selected according to user requirements in order to obtain fractions having required mean particle sizes.

The sieving means may be any means of the mentioned type used in the art. Preferably, sieving means comprising vibrating screens, where, due to the vibrations (having frequency set by the user) the particles move on the screen surface and are effectively separated into fractions, are used.

Typically, first screen serves for separation of fraction comprising particles of the largest size, usually having mean size corresponding to the size of particles fed in the drying step (e.g. in preferred embodiment mentioned above, these would be the particles having mean particle size equal to about 5 mm). After separating, this fraction is transported to the subsequent step of the method - finishing treatment step.

Subsequent screens are used for separating fractions, which may constitute a product ready for packing, but alternatively each of this fractions may also be directed to the finishing treatment step. These fractions may contain particles having required mean size typically in the range of from about 250 µm to about 1 mm, for example having mean size equal to about 250, 275, 300, 325, 350, 375, 400, 425, 450, 475, 500, 525, 550, 575, 600, 625, 650, 675, 700, 725, 750, 775, 800, 825, 850, 875, 900, 925, 950 and 1000 µm.

In a preferred embodiment of the invention, the sieving step is conducted using three levels of screens (having mesh size equal to about 5 mm, about 500 µm and about 250 µm), separating the dried saponite into three fractions:
- fraction 1 comprising particles having mean size equal to about 250 µm,
- fraction 2 comprising particles having mean size equal to about 500 µm,
- fraction 3 comprising particles having mean size equal to about 5 mm.

In this embodiment, fraction 3 is directed to the finishing treatment step, while fractions 1 and 2 are finished products ready for packing.

The mean particle size in each fraction may be verified using any measuring technique known in the art.

### Step E - Finishing treatment

After the sieving step, the selected fractions are transported to the finishing treatment step using transporting means. The transporting means may be any devices known in the prior art, which are used to transport loose raw material between different steps of technological processes. For example, such transporting means may, among others, include devices such as feeders, conveyors, e.g. belt conveyors, screw conveyors, dispensers etc., as well as any combination of such devices. A person skilled in the art would easily select a set of devices adapted to the given process conditions (e.g. available spatial conditions, type of devices used to treat the raw material in the finishing treatment step, user requirements, etc.).

The last step of the method of the invention is conducted in order to obtain saponite fraction comprising particles of very small mean particle size, impossible to obtain in the sieving step using mentioned separating means, and which according to the experiments conducted by the present inventors exhibit properties significantly more powerful than properties of fractions comprising particles of larger mean size.

Usually, in this step saponite fractions having mean particle size in the range of from 0,6 µm to 100 µm, preferably having mean particle size equal to about 0,6 µm, about 0,8 µm, about 1 µm, about 5 µm, about 10 µm, about 15µm, about 20 µm, about 25 µm, about 30 µm, about 40 µm, about 45 µm, about 50 µm, about 55 µm, about 60 µm, about 65 µm, about 70 µm, about 75 µm, about 80 µm, about 85 µm, about 90 µm, about 95 µm and about 100 µm, most preferably about 1 µm, about 20 µm, about 50 µm, about 80 µm, about 100 µm, are obtained.

The finishing treatment step may be conducted using any device known in the art, allowing for obtaining mean sizes of saponite grains as defined above.

In the preferred embodiment of the invention, the finishing treatment step is conducted in so called dispergator having construction developed by the present inventors. The device contains a drum comprising a shaft with seated discs, and milling blades located between the discs. The particles fed to the dispergator are put in a whirl motion by the rotating discs and are simultaneously milled by the rotating blades. There is also a set of screens located inside the drum, which separate the grinded particles into fractions. The set of screens contains a number of screens selected according to user requirements in order to obtain required number of fractions. Mesh sizes of the screens are selected according to user requirements in order to obtain fractions having required mean particle size. In a preferred embodiment, the set of screens contains three screens having mesh size equal to about 100 µm, about 50 µm and about 20 µm, respectively. In another preferred embodiment, the set of screens contains three screens having mesh size equal to about 80 µm, about 30 µm and about 1 µm, respectively.

In another preferred embodiment of the invention, it is possible, if required, to obtain during the finishing treatment step the saponite particles having mean particle size in nanometre range. Cyclone separators in connection with bag filters are used for this purpose. Preferably, in this embodiment of the invention at least one cyclone separator, and more preferably three of such devices are used. They allow for separation of at least one additional fraction of the modified saponite, and in a preferred embodiment three of such additional fractions. This at least one cyclone separator is connected via appropriate conduits with the device, in which the finishing treatment step is conducted, preferably with the dispergator as described above. Additionally, the set of cyclone separators is used in order to prevent the contamination of the air with the dust from the installation.

The saponite fractions obtained during the finishing treatment step constitute a finished product - described herein as "modified saponite of the invention".

After the finishing treatment step, the modified saponite of the invention is transported to the packing step using transporting means. The transporting means may be any devices known in the prior art, which are used to transport loose raw material between different steps of technological processes. For example, such transporting means may, among others, include devices such as feeders, conveyors, e.g. belt conveyors, screw conveyors, dispensers, charging hoppers etc., as well as any combination of such devices. A person skilled in the art would easily select a set of devices adapted to the given process conditions (e.g. available spatial conditions, user requirements, etc.).

### Fractions of the modified saponite

Depending on the selection of the process parameters in the sieving step (step D) and finishing treatment step (step E), one can obtain various fractions of the modified saponite of the invention characterized by various mean grain sizes (diameters). Fractions having various grain sizes differ one from another by their applications and activity, wherein the experiments conducted by the present inventors show, that the smaller the grain size is, the greater the activity.

For example, the fraction having smaller mean particle size (e.g. 80 µm) is recommended for use in washing cosmetic products, while the fractions having larger mean particle size (e.g. about 1 mm) are intended for use in peeling formulations or formulations, which are designed to remain on the skin for a longer time.

### Combination of the invention

According to the invention, in practical applications one may use not only the modified saponite of the invention, but also a combination comprising the modified saponite and the inter-packet water of the invention. As shown in the experiments conducted by the inventors, use of such a combination may result in obtaining additional advantageous technical effects (e.g. caring or therapeutic effects) due to unexpected properties of the activated inter-packet water of the invention, which exhibit better properties in terms of effectiveness of action with respect to human body and biological subjects in comparison to magnetically activated water.

Notwithstanding the above, the inter-packet water of the invention is an essential product of the method according to the invention, which may also have other applications than the ones cited below.

### Use of the modified saponite of the invention/the combination comprising the modified saponite and the inter-packet water of the invention.

The modified saponite of the invention or the combination comprising the modified saponite and the inter-packet water of the invention can be widely used in many fields of technology, including cosmetology, medicine and pharmacy, agriculture, food industry and other industries (e.g. chemical industry, paper industry, perfume industry, ceramic industry, paint industry, oil industry, hydroelectric power industry, construction industry, mining industry, e.g. as an agent for iron ore granulation, metallurgical industry, foundry industry, drilling industry, etc.). The materials can be also used as sorption materials.

The present invention provides a method for producing the modified saponite, which enables intensification of the properties of a natural saponite, so that the products comprising the modified saponite of the invention exhibit stronger action with the same dose or the same activity with smaller dose of the modified saponite, than analogous formulations comprising natural saponite.

### Cosmetics products

The natural saponite is known for its cosmetic activity and use in formulations for cosmetic purposes. The modified saponite of the invention and the combination comprising the modified saponite and the inter-packet water of the invention may be successfully used in analogous manner as the natural saponite, exhibiting stronger activity which enables using them in lower doses than the ones known in the prior art for the natural saponite.

The cosmetic formulation can have any form - typical for its intended use. In case of formulations intended for the skin such forms will, for example, include balsams, lotions, masks, bubble baths, soaps, gels, peelings, creams, milks, etc. In case of formulations used for hair, such forms will, for example, include shampoos, conditioners, foams, masks, pomades, waxes, creams, oils, gels, paints, hair sera, etc.

Besides development of the method for producing the modified saponite of the invention, the present inventors designed also a series of cosmetic formulations, which exhibit unexpectedly effective action. Several embodiments of such a formulation are described below in the examples of the invention.

Cosmetic products such as shower/bath gel, shower peeling, anti-dandruff shampoo, mask, usually employ the modified saponite of the invention or the combination comprising the modified saponite and the inter-packet water of the invention, mixed with cosmetic base. Such base may be any cosmetic detergent or formulation used for hair and/or body care. For example, such base may be a liquid soap, shampoo, shower gel, etc. Preferably, formulations for children are used due to the fact that their cosmetic effect is usually milder for the skin and/or hair because the composition for this type of products is selected with greater care in relation to irritating and allergenic properties.

If in the cosmetic formulations (e.g. the ones disclosed in the examples) additional auxiliary components are required, they may consist of any components of the indicated type. For example, if the formulation contains olive oil, it may be replaced with any liquid oil that is approved for use on the skin, e.g. sunflower oil, rapeseed oil, grapeseed oil, etc. Analogously, if honey is indicated in the composition of the formulation, it can be any kind of honey, adapted for example to the preferences of the user.

Typically, the content of the modified saponite of the invention or the combination comprising the modified saponite and the inter-packet water of the invention in formulations intended for cosmetic use is in range of from about 15 to about 97 %, preferably from about 15 to about 90%, more preferably from about 20 to about 80 %, for example from about 30 to about 70 % or from 40 to about 60% or from about 45 to about 55 %, and even equals 95-97%.

### Therapeutic products

The natural saponite is also known for its therapeutic activity. The modified saponite of the invention and the combination comprising the modified saponite and the inter-packet water of the invention may be successfully used for every indication for which the natural saponite is used, exhibiting stronger activity which makes it possible to use them in lower doses than the ones known in the prior art for the natural saponite.

In general, indications for use of the modified saponite of the invention and the combination comprising the modified saponite and the inter-packet water of the invention include, *inter alia,* generalized psychosomatic exhaustion, fatigue, asthenic conditions, cardiovascular disorders, lymph circulation disorders, removal of toxins from the body, arthritis of various aetiologies, inflammatory skin conditions of various aetiologies, allergies and diatheses, inflammatory gynaecological diseases, diabetes, nervous system disorders (including neurovegetative dystonia), respiratory disorders (including bronchial asthma), urological disorders (including prostate gland inflammation, adenoma, kidney stones, impotence, cystitis, varicocele, hydrocele, epididymo-orchitis, hypogonadism, warts, urethritis, etc.), orthopaedic disorders (including all conditions requiring relief from inflammation and oedema, e.g. bursitis, osteoarthritis, backache, rheumatoid arthritis, Achilles bursitis, intra-articular hematoma, trochanter, etc.), skin disorders (including psoriasis), radiculitis.

Especially important field, in which the modified saponite of the invention or the combination comprising the modified saponite and the inter-packet water of the invention can be used, is balneology.

In case of application on the skin, places of occurrence of irritations and inflammations should change colour, gradually adopting the colour of healthy skin. Itching, inflammations and skin blemishes fade away. In case of long term administration, the modified saponite of the invention and the combination comprising the modified saponite and the inter-packet water of the invention improve peripheral vasculature and improve circulation by normalizing blood pressure, normalize blood sugar level, increase potency, restore skin elasticity, improve prostate, pancreas, kidneys, liver and intestines activity, and additionally improve appetite. In addition, they eliminate susceptibility to pathogenic agents, reducing the incidence of influenza, tuberculosis, fungal infections and digestive system diseases.

According to the above, the modified saponite of the invention and the combination comprising the modified saponite and the inter-packet water of the invention may be used as a base for intestinal sorbents indented for removing heavy metal salts, radionuclides and pathogenic microbes (viruses (e.g. polio viruses, type A hepatitis viruses), bacteria (e.g. *Escherichia coli,* staphylococci)) and others from human body. The effectiveness of saponite acting as intestinal sorbent is higher than the activity of activated carbon which may damage the surface of digestive tract mucosa and which contains synthetic contaminants (benzo-a-piren and dioxins).

Furthermore, the modified saponite of the invention and the combination comprising the modified saponite and the inter-packet water of the invention may be used in the treatment of many disease related to metabolic disorders.

Additionally, according to the conducted experiments, the modified saponite of the invention and the combination comprising the modified saponite and the inter-packet water of the invention exhibit unexpected therapeutic properties with respect to skin diseases, especially psoriasis.

According to the conducted experiments, the modified saponite of the invention and the combination comprising the modified saponite and the inter-packet water of the invention can also be used to treat mouth cavity disease, e.g. gums diseases.

Similarly as the natural saponite, the modified saponite of the invention and the combination comprising the modified saponite and the inter-packet water of the invention can be used in the treatment of the alcohol withdrawal syndrome.

In case of most indications, dose of the modified saponite of the invention and the combination comprising the modified saponite and the inter-packet water of the invention should be in the range of 15-20 g, administered orally 1-3 times per day, preferably 2-3 times per day.

The therapeutic formulation comprising the modified saponite can have any form depending mainly on the administration route. In case of formulations intended for oral administration, the therapeutic formulation may be in the form of powder or granulate, intended for preparing suspension in water (e.g. placed in a sachet containing one dose of the modified saponite), dispersible tablets, coated and uncoated tablets, pills, capsules, as well as suspensions, etc.

In case of formulations intended for the administration on the skin, in particular for transdermal administration, such forms will, for example, include balsams, lotions, emulsions, creams, ointments, gels, pastes, powders, foams, lipsticks, drops, sprays and suspensions, etc.

In any case, the therapeutic formulation may contain at least one auxiliary component, typical for a given dosage form, facilitating the processing of the modified saponite of the invention or the combination of the invention into a formulation. Such auxiliary agents include, *inter alia,* diluents, excipients, carriers, fillers (e.g. sugars, cellulose formulations, calcium phosphate, etc.), binders (e.g., starch, gelatin, tragacanth, methylcellulose, hydroxypropylmethylcellulose, polyvinylpyrrolidone, etc.), disintegrants (e.g., starch, cross-linked polyvinylpyrrolidone, alginic acid, etc.), lubricants (e.g., talc, stearic acid and salts thereof, etc.), glidants, sweeteners, fragrances, preservatives, stabilizers, wetting agents, emulsifiers, antioxidants, buffering agents, natural ingredients, etc. The therapeutic formulation may also contain auxiliary agents not mentioned above.

Additionally, besides the modified saponite of the invention or the combination of the invention, the therapeutic formulation may contain other biologically active agents, enhancing the action of the modified saponite of the invention or providing an additional therapeutic effect. Such additional active agent may be e.g. an antiviral agent, antibacterial agent, antifungal agent, antiemetic agent, cholesterol-lowering medicine, herbal ingredient, activated carbon, etc. The selection of amount of the additional active agent depends on its type and form of the therapeutic formulation. The amount can thus be readily selected by the skilled person on the basis of the prior art and generally available knowledge in the art.

Therapeutic formulations comprising the modified saponite of the invention or the combination of the invention may be prepared using any method known in the field of pharmacy, for example, using traditional blending, granulating, tablet- and dragee-preparation techniques, dissolving, lyophilizing, etc. The selection of appropriate techniques will depend on the targeted form of the therapeutic formulation.

Content of the modified saponite of the invention or the combination comprising the modified saponite and inter-packet water of the invention in therapeutic formulations is usually in the range from about 15 to about 97 %, preferably from about 15 to about 90%, more preferably from about 20 to about 80 %, for example from about 30 to about 70 % or from about 40 to about 60%, or from about 45 to about 55 %, and even is equal to 95-97%.

Analogously to the cosmetic formulations, present inventors also developed series of formulations having therapeutic activity, using the modified saponite obtained by the method of the invention. Several such formulations are described below in Examples of the invention.

### Agricultural products

The invention can be also widely used in agriculture. In particular it may be used as a mineral feed (e.g. for pigs, young cattle, sheep, camels, poultry, furred animals (e.g. rabbits), fish, etc.), a feed additive, a feed preservative, a premix, a mineral and vitamin supplement for animals (e.g. for animals mentioned above, but also for pets e.g. dogs, cats, rodents, birds, etc.), a crop support, a plant protection product, a crop preservative, a soil conditioner, a fertilizer (including agricultural fertilizers, mineral fertilizers, as well as fertilizers for pot plants), a soil deoxidizer, etc.

The modified saponite of the invention will exhibit activity similar to the activity of the natural saponite as described in literature, whereby due to the modification obtained by the method of the invention and optional use of the mixture comprising the modified saponite and inter-packet water, a intensified activity is obtained, which allows for decreasing the amount of the modified saponite necessary for obtaining the same technical effect.

Content of the modified saponite of the invention or the combination comprising the modified saponite and inter-packet water of the invention in agricultural formulations is usually in the range from about 10 to about 100 % by weight, preferably from about 20 to about 90% by weight, more preferably from about 30 to about 80 % by weight, for example from about 40 to about 70 % by weight or about 50 to about 60% by weight, or from about 45 to about 55 % by weight, and is even equal to 100% by weight.

Recommended doses of the modified saponite or the combination comprising the modified saponite and inter-packet water of the invention in case of breeding applications are:
- poultry feed - about 0,07 - about 1,0% by weight of the modified saponite/the combination comprising the modified saponite and inter-packet water with respect to the mass of the feed,
- rabbit feed - about 1,0% by weight of the modified saponite/the combination comprising the modified saponite and inter-packet water with respect to the mass of the feed,
- cattle feed - about 5 - about 10% by weight of the modified saponite/the combination comprising the modified saponite and inter-packet water with respect to the mass of the feed.

In case of employing the invention with respect to cultivation (e.g. as a crop support, a plant protection product, a crop preservative, a soil conditioner, a fertilizer, a mineral fertilizer, a soil deoxidizer, etc.) products containing even up to 100% of the modified saponite/the combination comprising the modified saponite and inter-packet water can be used.

### Sorption products

The natural saponite exhibit very effective sorption properties used, *inter alia,* for purification of water from various contaminants, in filter constructions, etc. The modified saponite of the invention exhibit the same properties and may be used in every application in which the natural saponite and other clay minerals having similar properties (e.g. smectites) are used. Thus, modified saponite of the invention may be, *inter alia,* used as a component of adsorbents used for purifying liquid organic media (edible oils, wines, juices, alcohol beverages, milk and others) for improving the quality and for regeneration of fuels and greases, as an agent for purifying chemical industry wastes, as well as for purifying potable water from contamination admixtures, etc.

Modification of the natural saponite using the method of the invention results in improving mentioned sorption properties, so the effectiveness of methods or devices employing the modified saponite of the invention increases. Thus, it is possible e.g. to conduct sorption processes faster and with increased throughput.

### Food industry

Besides using saponite as a sorption agent e.g. in processes for manufacturing beverages, advantageous therapeutic and prophylactic properties of the modified saponite may be also used in food industry. Addition of the modified saponite of the invention or the combination comprising the modified saponite and inter-packet water of the invention may therefore be used in food products such as, amongst others, bakery and confectionery products. Thus, the advantageous properties of the modified saponite of the invention or the combination comprising the modified saponite and inter-packet water of the invention may be employed in food products consumed every day.

The content of the modified saponite of the invention or the combination comprising the modified saponite and inter-packet water of the invention in food products is usually much lower than the amount used in case of cosmetics or therapeutic formulations, and typically is in the range from about 0,01 to about 0,1% by weight, preferably from about 0,02 to about 0,09 % by weight, more preferably from about 0,03 to about 0,06 % by weight, especially preferably from about 0,04 to about 0,05 % by weight, and most preferably in amount equal to 0,07% by weight.

### Construction industry

According to the experiments conducted by the present inventors, the modified saponite of the invention may be used in applications, in which similar mineral materials, e.g. smectites or bentonite, are used. Thus, for example the modified saponite of the invention may be used in manufacturing of construction materials (i.a. thermal isolation, waterproofing, fillers and others). An especially preferred example is manufacturing of LECA based on saponite clay. A material having promising properties e.g. having an inherent density up to 50 kg/m3, was obtained while maintaining low production costs.

According to the invention the term "about" as used hereinabove and hereinbelow, should be understood as +/- 5% deviation from a given value, reflecting the inaccuracies that may occur during the course of the method according to the invention.

### Examples

### Example 1. Production of the modified saponite (fractions: about 500 µm, about 250 µm, about 100 µm, about 50 µm and about 20 µm)

The starting material is a saponite coming directly from deposits, comprising portions having mean size from about 3 to about 50 cm, drawn out using an excavator. The saponite was transported from a storage to the processing location.

The raw saponite was introduced portionwise to a charging hopper connected with the inlet of a crusher - in the example of the method according to the invention for that purpose a crusher comprising two rotors with toothed crushing discs arranged on shafts in such manner that distance between crushing discs, engaging with each other, was about 5 mm, was used. As a result, initially comminuted saponite leaving the crusher comprised particles having maximum size equal to about 5 mm.

The initially comminuted saponite was transported by belt conveyor to the inlet of a drum dryer. Inside of the drum dryer, the initially comminuted saponite was heated in the temperature of 65°C for 2 hours. An external boiler fired with coal, oil or gas connected with the dyer via conduits transporting hot air was used as a heat energy source for drying the saponite. The hot air from the boiler heats the heating plates arranged inside the dryer. The uniform distribution of the hot air emitted by the heating plates is achieved by the use of a fan providing a stream of hot drying air moving in the same direction as the saponite subjected to drying.

During drying, the saponite is displaced inside the dryer using a screw conveyor. The particles of saponite transported using the screw conveyor interact mutually in a frictional manner, so that during drying the secondary comminution process occurs. In order to achieve additional comminuting effect, the dryer is equipped with friction elements in the form of plates having "L"-like profile, disposed on the internal wall of the drum dryer.

Besides the drying and the secondary comminution, process of evaporation of the inter-packet water occurs in the dryer. The steam moving together with the hot air stream was removed from the dryer using conduit located on the top of the dryer. The steam was transported via said conduit to the condenser, and subsequently the condensed water was directed to an external reservoir.

In order to separate optional dust particles and prevent the contamination of environment, the air leaving the drum dryer goes through a conduit to a cyclone separator connected to a bag filter.

The dried saponite leaving the dryer contained particles having various sizes, whereby the maximum particle size was about 5 mm. The water content after completion of the drying step was from about 8 to about 10%.

The dried saponite was transported to the sieving step using a screw conveyor and a charging hopper. In the sieving step, the dried saponite was separated into 3 fractions using a set of three vibrating screens.

On the first, upper screen the fraction comprising particles of the largest size, having mean size of about 5 mm, was separated. After sieving, this fraction was transported as semi-finished product to the subsequent step of the method - the finishing treatment step.

On the second screen particles having mean size of about 500 µm were separated, while on the third one particles having mean size of about 250 µm were separated. Fractions separated in such a manner were transported using a screw conveyor to a packing station, where they were packed in bags capable of holding from 3 to 25 kg of the finished product.

As mentioned above, the fraction comprising particles having mean size of about 5 mm was transported using the screw conveyor to the finishing treatment step. This step of the method was conducted in an assembly comprising the dispergator and one set of cyclone separator and bag filter. The dispergator used in this step was equipped with a drum comprising a shaft with seated discs, with 280 milling blades located between the discs. The particles of saponite fed to the dispergator were entrained by an air rush generated by the rotating blades and, as a result of contact with the blades, their size was reduced. The air rush ensured also that the grinded particles were directed to the separating screens. The dispergator's drum was equipped with a set of three screens having mesh sizes equal to about 100 µm, about 50 µm and about 20 µm, respectively. As a result, the fractions leaving the dispergator had mean particle size of about 100 µm, about 50 µm and about 20 µm. The fractions were next directed to the packing station using a charging hopper and a screw conveyor.

The air leaving the dispergator was directed to the assembly comprised of a cyclone separator and a bag filter. In this embodiment of the method, finer fractions of the saponite were not separated - the assembly.

, comprising cyclone separator and bag filter served only to prevent the air contamination.

Throughput of the method of the invention conducted in the above described manner is about 2 tons per hour.

### Example 2. Production of the modified saponite (fractions: about 1 mm, about 300 µm, about 80 µm, about 30 µm and about 1 µm)

In the second embodiment of the invention, the process has been conducted in analogous manner as in Example 1, wherein:
- in the sieving step screens having mesh sizes of about 5 mm, about 1 mm and about 300 µm, were used, and
- in the finishing treatment step, screens having mesh sizes of about 80 µm, about 30 µm and about 100 µm, were used
as a result, giving fractions of the modified saponite having mean grain size equal to about 1 mm, about 300 µm, about 80 µm, about 30 µm and about 100 µm.

### Example 3 - Shower/bath gel

Using the products obtained by the method of the invention shower/bath gel was prepared, containing:
450 ml of liquid soap for children,
50 ml of inter-packet water of the invention,
50 g of the modified saponite (fraction having mean particle size - 80 µm - Example 2).

### Use:

Typically, a dose of 400-450 ml is used for a single bath/application. The gel is used in several steps. In the first phase of application of the formulation, the duration of the bath should not exceed about 10-12 minutes. The first phase includes 3 applications. In the second phase, duration of the application/bath may be gradually increased to about 25-30 minutes. In this step, if there are no undesired effects, the number of applications may be adapted to the user's needs. After each bath, skin needs to be washed with water. If the undesired effects occur, it is recommended to lower the dose to about 200-220 ml.

### Example 4 - Shower peeling

Using the products obtained by the method of the invention shower peeling was prepared, containing:
450 ml of liquid soap for children,
50 ml of inter-packet water of the invention,
50 g of the modified saponite (fraction having mean particle size - 1 mm - Example 2).

### Example 5 - Anti-dandruff shampoo

Using the products obtained by the method of the invention anti-dandruff shampoo was prepared, containing:
300 ml of liquid soap for children,
100 ml of inter-packet water of the invention,
100 g of the modified saponite (fraction having mean particle size - 80 µm - Example 2).

### Use:

Usually, a dose of 40-50 ml is used for an application. After applying the formulation on hair it is recommended to massage the skin for period of 3-5 minutes, and then to wash the head using hot/warm water and leave the hair/skin to dry. If needed, the treatment may be repeated two times. The use of the shampoo of the example does not require using additional soap and other detergents.

### Example 6 - Cosmetic mask

Using the products obtained by the method of the invention cosmetic mask was prepared, containing:
300 ml of liquid soap for children,
15-20 g of the modified saponite (fraction having mean particle size - 1 mm - Example 2).
150-200 ml of inter-packet water of the invention,
5 ml of olive oil.

### Use:

The mask should be gently applied to the face and left for period of about 20-30 minutes. Then, it should be washed with warm water.

### Example 7 - Formulation

For this formulation it is necessary to open the skin pores in order to facilitate the penetration of the formulation into the skin. For example it may be achieved by using a hot bath three times. Then the whole body should be lubricated with the formulation of given composition, and it should be left on the skin for 20 minutes and washed with water without detergents.

### Example 8 - Formulation for caring/treating gums

Using the products obtained by the method of the invention, formulation for the use on gums was prepared, containing:
15-20 g of the modified saponite (fraction having mean particle size - 1 mm - Example 2).
45-80 ml of inter-packet water - to obtain the consistency of sour cream.

### Use:

The composition needs to be impregnated in a sterile manner. Next, thus impregnated swabs should be placed on gums for maximum period of 15 minutes. Then, the mouth should be washed with water.

## Claims

1. A method for producing modified saponite **characterized in that** it comprises steps of:
a. providing a raw saponite,
b. initial comminution of the raw saponite,
c. drying of the initially comminuted saponite with simultaneous secondary comminution, and inter-packet water separation in the temperature up to 65°C,
d. sieving a dried saponite using at least one screen to obtain at least two fractions,
e. finishing treatment to obtain at least one fraction of modified saponite having mean particle size in the range of from about 0,6 µm to about 100 µm.

2. The method according to any of the previous claims **characterized in that** the drying step is conducted in a drum dryer, in which the moving saponite undergoes abrasive treatment due to the mutual friction between saponite particles, and optionally due to the friction against friction elements located inside the drum of the dryer.

3. The method according to any of the previous claims **characterized in that** during step c), a drying stream of hot air is used, flowing co-currently or counter currently with respect to the direction of the flow of the dried saponite.

4. The method according to any of the previous claims **characterized in that** during step c), evaporated inter-packet water is discharged from the drying device.

5. The method according to any of the previous claims **characterized in that** during step d) a set comprising at least three screens is used, providing at least three fractions having mean particle sizes of less than about 250 µm, less than about 500 µm and less than about 5 mm, respectively or
a set comprising at least three screens is used, providing at least three fractions having mean particle sizes of less than about 300 µm, less than about 1000 µm and less than about 5 mm, respectively.

6. The method according to any of the previous claims **characterized in that** only one fraction obtained in step d) having the largest mean particle size, is directed to step e).

7. The method according to any of the previous claims **characterized in that** dried, sieved raw material is further grinded and separated in step e) resulting in obtaining at least two fractions.

8. The method according to any of the previous claims **characterized in that** the dried, sieved raw material undergoes a finishing treatment in step e) using an assembly comprising a cyclone separator and a bag filter, to obtain a saponite fraction having mean particle size in nanometer range.

9. A modified saponite obtained by the method as defined in one of the claims 1-8.

10. An inter-packet water obtained in the step c) of the method as defined in one of the claims 1-8.

11. A combination comprising the modified saponite as defined in claim 9 and the inter-packet water as defined in claim 10.

12. A combination according to claim 11 **characterized in that** it comprises 0,1-99,9 % of the modified saponite and 0,1 - 99,9% of the inter-packet water.

13. Use of the modified saponite as defined in claim 9 or the combination as defined in claim 11 or 12 as a therapeutic product, a cosmetic product, a food product, an agricultural product, a sorption product or a construction product.

14. A product comprising the modified saponite as defined in claim 9 or the combination as defined in claim 11 or 12 and, optionally, at least one additional active component and/or auxiliary components.

15. The product according to claim 14 **characterized in that** it is a therapeutic product, a cosmetic product, a food product, an agricultural product, a sorption product or a construction product.
